(19) Europäisches Patentamt
European Patent Office
Office européen des brevets

(11) EP 3 723 030 A1

(12) EUROPEAN PATENT APPLICATION
published in accordance with Art. 153(4) EPC

(43) Date of publication:
14.10.2020 Bulletin 2020/42

(51) Int Cl.:
G06Q 50/22 (2018.01)

(21) Application number: 17933918.9

(22) Date of filing: 05.12.2017

(86) International application number:
PCT/JP2017/043670

(87) International publication number:
WO 2019/111327 (13.06.2019 Gazette 2019/24)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD TN

(71) Applicant: DSI Corporation
Tokyo 104-0061 (JP)

(72) Inventor: SATO Hiroshi
Tokyo 104-0061 (JP)

(74) Representative: Granleese, Rhian Jane
Marks & Clerk LLP
15 Fetter Lane
London EC4A 1BW (GB)

(54) TECHNICAL FEE AUTOMATIC CALCULATION SYSTEM, TECHNICAL FEE AUTOMATIC CALCULATION METHOD, AND PROGRAM

(57) Provided are a dental technical fee automatic calculation system, a dental technical fee automatic calculation method, and a program capable of estimating a basis of billing or a billing amount from an image of a prosthesis. The dental technical fee automatic calculation system 100 is provided with an image data input unit 110 configured to input image data of the prosthesis, a basis data input unit 120 configured to input basis data as a basis of assessment of a billing amount for the prosthesis, and a learning unit 130 to which the image data and the basis data are input to construct a learning model indicative of a correlation between the image data and the basis data.

FIG. 1

EP 3 723 030 A1

**Description**

**[Technical Field]**

The present invention relates to a dental technical fee automatic calculation system, a dental technical fee automatic calculation method, and a program, and more particularly, to a technology for estimating a basis of billing or a billing amount from an image of a prosthesis.

**[Background Art]**

Conventionally, a labo slip is used for ordering and order reception between a dental clinic and a dental laboratory. The labo slip is a document issued by the dental clinic and is loaded with, for example, the name of a patient, name and quantity of a dental technical product, specifications (capable of including instructions related to a material used, creation method, etc.) of the dental technical product, names of the dental clinic as an order source and the dental laboratory as a supplier, and the like. In general, the dental clinic creates the labo slip by filling out an existing labo slip form with necessary items by handwriting.
Patent Literature 1 describes a computer system capable of performing ordering and order reception of prostheses (equivalent to the dental technical products) between a plurality of dental clinics and a plurality of dental laboratories.

**[Citation List]**

**[Patent Literature]**

**[Patent Literature 1]** Japanese Patent Application Laid-Open No. 2016-071784

**[Summary of the Invention]**

**[Problem to be Solved by the Invention]**

The system described in Patent Literature 1 improves the convenience by computerizing the conventional paper-based business for ordering and order reception. A dental laboratory creates a prosthesis based on a labo slip issued by a system, such as that described in Patent Literature 1, or handwritten in an existing form. For the finished prosthesis, a billing amount is calculated based on the name and quantity of the prosthesis, type and quantity of the material used, creation method, and the like (hereinafter referred to as the basis of billing) and a dental clinic is charged for it.
The basis of billing or the billing amount depends exclusively on a self-declaration of the dental laboratory. If the dental laboratory can keep a record related to a specific material used and processes of work, the basis of billing or the billing amount can be calculated based on the record, but it is not easy. Although the basis of billing or the billing amount can be calculated based on the labo slip, this method is not necessarily appropriate because the prosthesis may sometimes be created in consideration of an item or items that are not definitely described in the labo slip. Actually, therefore, the dental laboratory not infrequently counts a presumably reasonable basis of billing or billing amount based on experiences or the like, with reference to the finished prosthesis or its photograph.
However, it is difficult to objectively support the accuracy of the basis of billing or the billing amount calculated in this manner. The propriety of the basis of billing or the billing amount is a serious concern for each of the dental laboratory, the dental clinic, an audit institution, and the like. Accordingly, there is a demand for the provision of a means for objectively supporting the propriety of the basis of billing or the billing amount for each prosthesis.
The present invention has been made to solve such a problem, and its object is to provide a dental technical fee automatic calculation system, a dental technical fee automatic calculation method, and a program capable of estimating a basis of billing or a billing amount from an image of a prosthesis.

**[Arrangement for Solving the Problem]**

A dental technical fee automatic calculation system according to one embodiment of the present invention comprises an image data input unit configured to input image data of a prosthesis, a basis data input unit configured to input basis data as a basis of assessment of a billing amount for the prosthesis, and a learning unit to which the image data and the basis data are input to construct a learning model indicative of a correlation between the image data and the basis data.

In the dental technical fee automatic calculation system according to the one embodiment of the present invention, the learning unit receives the input of the image data and outputs the basis data highly correlated to the image data based on the learning model, and the system further comprises a billing amount estimation unit configured to estimate the billing amount based on the basis data.

In the dental technical fee automatic calculation system according to the one embodiment of the present invention, the image data input unit inputs the image data of the prosthesis and image data related to a labo slip, and the learning unit updates the learning model using the contents of the labo slip if the basis data output by the learning unit based on the image data of the basis data and the contents of the labo slip are different.

In the dental technical fee automatic calculation system according to the one embodiment of the present invention, the image data input unit inputs the image data of the prosthesis and image data related to a labo slip, and the billing amount estimation unit outputs the different items.

A dental technical fee automatic calculation method according to one embodiment of the present invention comprises an image data input step in which a computer inputs image data of a prosthesis, a basis data input step for inputting basis data as a basis of assessment of a billing amount for the prosthesis, and a learning step in which the image data and the basis data are input to construct a learning model indicative of a correlation between the image data and the basis data.

In the dental technical fee automatic calculation method according to the one embodiment of the present invention, the image data is input and the basis data highly correlated to the image data is output based on the learning model in the learning step, and the billing amount is estimated based on the basis data.

In the dental technical fee automatic calculation method according to the one embodiment of the present invention, the image data of the prosthesis and image data related to a labo slip are input in the image data input step, and the method further comprises a step for updating the learning model using the contents of the labo slip if the basis data output based on the image data of the basis data in the learning step and the contents of the labo slip are different.

In the dental technical fee automatic calculation method according to the one embodiment of the present invention, image data of the prosthesis and image data related to a labo slip are input in the image data input step, and the method further comprises a step for outputting the different items if the basis data output based on the image data of the basis data in the learning step and the contents of the labo slip are different.

A program according to one embodiment of the present invention is a program for urging a computer to execute the above-described method.

A dental technical fee automatic calculation system according to one embodiment of the present invention is a dental technical fee automatic calculation system comprising an image data input unit configured to input image data of a prosthesis, a basis data input unit configured to input a billing amount for the prosthesis, and a learning unit to which the image data and the billing amount are input to construct a learning model indicative of a correlation between the image data and the billing amount.

**[Effects of the Invention]**

According to the present invention, there can be provided a dental technical fee automatic calculation system, a dental technical fee automatic calculation method, and a program capable of estimating a basis of billing or a billing amount from an image of a prosthesis.

**[Brief Description of Drawings]**

[FIG. 1] A block diagram showing a structure of a dental technical fee automatic calculation system 100.
[FIG. 2] A block diagram showing a structure of the dental technical fee automatic calculation system 100.
[FIG. 3] A flowchart showing an operation of a dental technical fee automatic calculation system 100 according to Example 1.
[FIG. 4] A diagram showing an example of a fee table.
[FIG. 5] A flowchart showing an operation of a dental technical fee automatic calculation system 100 according to Example 2.
[FIG. 6] A flowchart showing an operation of a dental technical fee automatic calculation system 100 according to Example 3.

**[Mode for Carrying Out the Invention]**

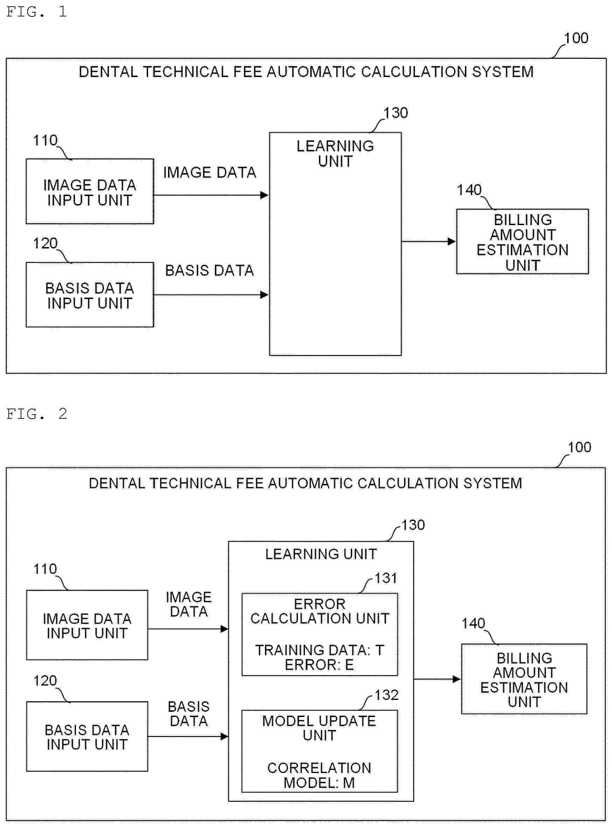

A specific embodiment to which the present invention is applied will now be described in detail with reference to the accompanying drawings. First, a structure of the dental technical fee automatic calculation system 100 according to the embodiment of the present invention will be described with reference to the block diagram of FIG. 1.

The dental technical fee automatic calculation system 100 is an information processor configured to independently learn a correlation between an image of a prosthesis and the basis of billing by machine learning. Typically, the dental technical fee automatic calculation system 100 is an information processing system that implements predetermined processing by executing software (learning algorithm, etc.) read out from a storage device by a central processing unit (CPU). The dental technical fee automatic calculation system 100 may be either composed of a single information processor or constructed by dispersive processing by a plurality of information processors.

The dental technical fee automatic calculation system 100 comprises an image data input unit 110 configured to acquire image data of the prosthesis, a basis data input unit 120 configured to acquire basis data indicative of a part of the basis of billing for the prosthesis, a learning unit 130 configured to learn the correlation between the image data and the basis data, and a billing amount estimation unit 140.

The image data input unit 110 may be either implemented by hardware (CPU, etc.) or logically implemented as hardware executes a function defined by software. Typically, the image data input to the image data input unit 110 is two-dimensional image data obtained by photographing the prosthesis created by a dental laboratory using a camera. Alternatively, three-dimensional image data obtained by adding depth information to two-dimensional image data or three-dimensional model data generated by means of a three-dimensional scanner or the like may be used as the image data.

The image data input unit 110 can extract a feature quantity from the input image data. Deep learning is a typical technique for feature quantity extraction from the image data. The deep learning is a machine learning technique using a multi-layer neural network. The deep learning is performed so that an output error is minimal when the input data is input to the multi-layer neural network, by using a technique called back propagation. In this way, the multi-layer neural network is adjusted so that the feature quantity of the input data can be extracted.

Moreover, the image data input unit 110 may input image data in a labo slip for the prosthesis together with the image data concerned. The image data of the prosthesis and the image data in the labo slip may be either different or identical (i.e., the prosthesis and the labo slip may be imprinted in a single image). The image data input unit 110 extracts items mentioned in the labo slip from the image data in the labo slip. For example, the image data input unit 110 can read a barcode, QR code, and the like mentioned in the labo slip, acquire identification information contained in the barcode, QR code, and the like, and use the identification information as a key to acquire information to be the basis of billing from a management system or the like for a labo slip (not shown). Alternatively, the image data input unit 110 may acquire information to be the basis of billing mentioned in the labo slip, by using a known technology such as OCR (optical character recognition). These pieces of information acquired from the labo slip can be used as basis data in a learning mode. Alternatively, they can be used to verify the propriety of the result of determination in a determination mode.

The basis data input unit 120 may be either implemented by hardware (CPU, etc.) or logically implemented as hardware executes a function defined by software. The basis data input to the basis data input unit 120 may include, for example, the type of the prosthesis (i.e., name of the prosthesis) and the quantity of the prosthesis included in the image data. In addition, the basis data may include the name of the material, used material quantity, creation method, and the like used in creating the prosthesis. More specifically, the basis data is one or a plurality of pieces of information constituting the basis of the billing.

Furthermore, depending on the business usage or the like, the basis data constituting the basis of billing may sometimes vary with every prosthesis type. If the prosthesis type is a "false tooth", for example, the used material quantity is not used as the basis of billing. In contrast, the used material quantity may sometimes be used as the basis of billing for another prosthesis type. In order to cope with such a case, the basis data input unit 120 may have a function of outputting only necessary basis data to the learning unit 130. For example, the basis data input unit 120 is provided with a table in which the prosthesis type and the necessary basis data are associated. The basis data input unit 120 can output only the basis data corresponding to the prosthesis type with reference to the table concerned when the basis data is input.

The learning unit 130 may be either implemented by hardware (CPU, etc.) or logically implemented as hardware executes a function defined by software. The learning unit 130 has a learning mode in which it learns the correlation between image data (hereinafter simply referred to as image data, although including a feature quantity of image data) and a determination mode in which it outputs basis data highly correlated to input image data using the result of learning in the learning mode.

In the learning mode, the learning unit 130 repeatedly receives input of various sets of image data and basis data and repeatedly executes learning processes. A learning model indicative of the correlation between the image data and the basis data is constructed by repeatedly executing the learning processes in this manner. The correlation indicated by the learning model gradually increases its reliability as the learning processes advance. When a learning model of a fully reliable level is constructed, the learning model concerned can be used to determine the basis data most highly correlated to the input image data.

FIG. 2 is a block diagram showing a structure of the dental technical fee automatic calculation system 100 comprising the learning unit 130 that performs supervised learning as a learning algorithm. The supervised learning is a technique for constructing the learning model by abundantly inputting data sets (hereinafter referred to as training data) composed of inputs and their corresponding outputs and identifying the correlation between the inputs and the outputs from the training data. Since the supervised learning is a known technology, although it can be implemented using a neural network, for example, a description of its detailed structure is omitted herein.

The learning unit 130 comprises an error calculation unit 131, configured to calculate an error E between a correlation model M derived from the image data and the basis data and a correlation feature identified from training data T provided in advance, and a model update unit 132 for updating the correlation model M so as to reduce the error E. The learning unit 130 goes on learning the correlation between the image data and the basis data as the model update unit 132 repeats the update of the correlation model M.

An initial value of the correlation model M represents a simplified (e.g., by a linear function) correlation between the image data and the basis data, for example, and is given to the learning unit 130 before the start of the supervised learning. The training data T is a data set of, for example, an image of a prosthesis created in the past and a basis of billing accurately recorded when the prosthesis concerned is created. The error calculation unit 131 identifies a correlation feature indicative of the correlation between the image data and the basis data from a lot of training data T given to the learning unit 130 and obtains the error E between this correlation feature and the correlation model M corresponding to the image data and the basis data in the current state. The model update unit 132 updates the correlation model M in a direction to reduce the error E according to a predetermined update rule. By repeating this process, the correlation model M is gradually adjusted so that it accurately indicates the correlation between the image data and the basis data.

In the determination mode, the learning unit 130 can automatically accurately obtain the basis data corresponding to the image data, based on the learning model constructed in the learning mode. More specifically, by giving the image data of the prosthesis as an input to the learning model, the learning model is enabled to automatically accurately output the basis of billing (name and quantity of the prosthesis, type and quantity of the material used, creation method, etc.).

The billing amount estimation unit 140 calculates the billing amount based on the basis of billing output by the learning unit 130 in the determination mode. For example, the billing amount estimation unit 140 has a fee table that defines the correspondence between the basis of billing and a unit cost of billing, a billing amount calculation rule, and the like. For example, the fee table may be one that defines a unit material cost per unit material quantity for each material name, one that defines a dental technical fee for each creation method, or one that defines a billing amount integration rule for each prosthesis type. The billing amount estimation unit 140 adds up the billing amount using the basis of billing output by the learning unit 130 and the description in the fee table.

The unit material cost, unit wage, and the like may sometimes fluctuate depending on the social situation. Also, the billing amount calculation rule and the like may sometimes be changed due to a modification in law or the like. Also in such a case, according to the present embodiment, a correct billing amount can continue to be calculated by modifying the description in the fee table. More specifically, it is unnecessary to execute the learning process again to re-create the learning model.

Some examples of execution will now be disclosed for a method of utilizing the learning model created in the learning process described above.

<Example 1>

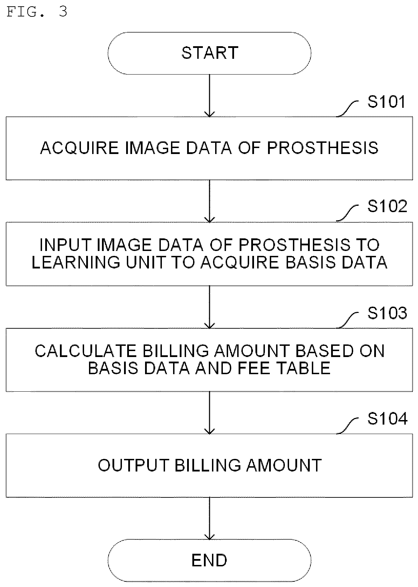

Example 1 relates to a dental technical fee automatic calculation system 100 for automatically calculating a billing amount related to a prosthesis using a learning model. An operation of the dental technical fee automatic calculation system 100 according to Example 1 will be described with reference to the flowchart of FIG. 3.

S101: An image data input unit 110 acquires image data of the prosthesis. For example, a dental technician photographs the prosthesis created for him/herself by using a smart phone provided with a camera as a constituent element of the image data input unit 110. The image data input unit 110 extracts a feature quantity from the image data.

S102: The image data input unit 110 inputs the feature quantity of the image data acquired in S101 to the learning unit 130. The learning unit 130 inputs the feature quantity of the image data to the learning model and obtains, as an output, basis data highly correlated to the image data. The basis data obtained here includes, for example, the type of the prosthesis (i.e., name of the prosthesis), quantity of the prosthesis, name of the material used, used material quantity, and the like.

S103: A billing amount estimation unit 140 assesses the billing amount based on the basis data obtained in S102 and a fee table retained in advance.

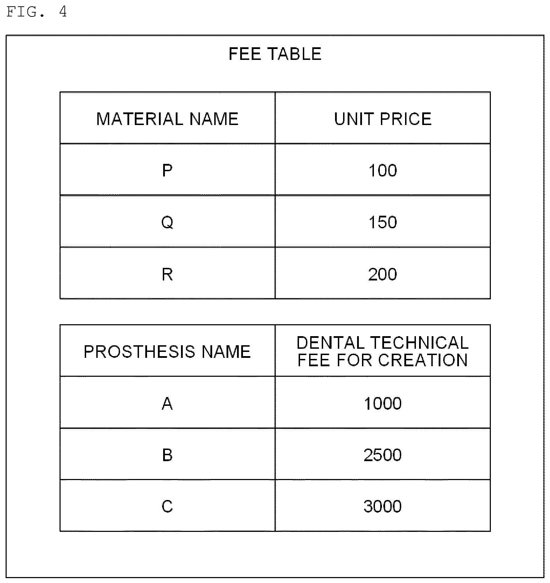

FIG. 4 shows an example of the fee table. In this fee table, a unit cost and a dental technical fee for creation are defined for each material name and each prosthesis type, respectively. In this case, the billing amount estimation unit 140 can assess the billing amount according to equation (1).

$$\text{Billing amount} = \text{Prosthesis quantity} \times (\text{Unit cost} \\ \text{for used material name} \times \text{Used material quantity} + \text{Dental} \\ \text{technical fee for creation of prosthesis type}). \\ \cdots (1)$$

For example, according to the basis data obtained in S102, the prosthesis quantity, prosthesis type, prosthesis quantity, name of material used, and used material quantity are assumed to be 1, A, 1, P, and 10, respectively. According to the fee table, moreover, the unit cost of the material P and the dental technical fee for creation of the prosthesis A are assumed to be 100 yen and 1,000 yen, respectively. In this case, the billing amount is given by:

$$1 \times (100 \text{ yen} \times 10 + 1,000 \text{ yen}) = 2,000 \text{ yen}.$$

S104: The billing amount estimation unit 140 outputs the billing amount assessed in S103. For example, the billing amount can be displayed on a display device (not shown). Alternatively, the billing amount can be provided for a billing system (not shown) to be used when the billing system issues a bill.

<Example 2>

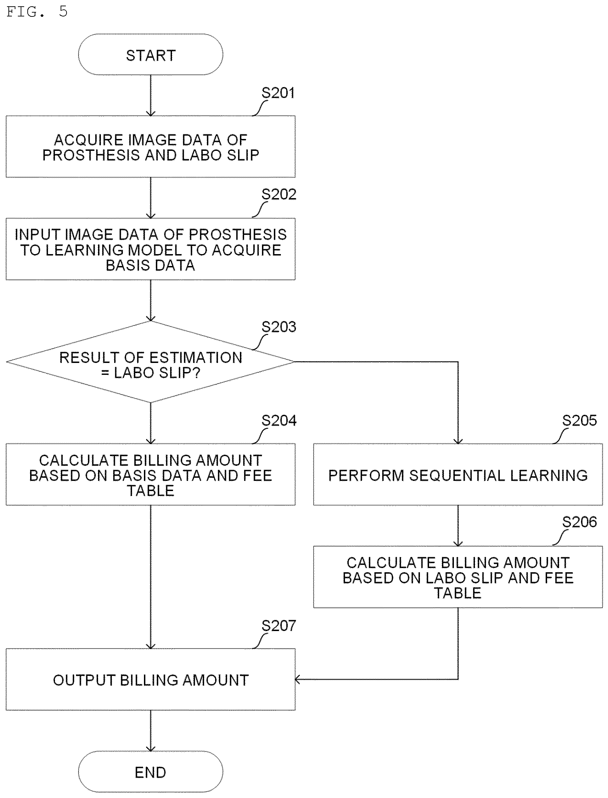

Example 2 relates to an automatic calculation system 100 capable of updating a learning model as required to continuously maintain and improve the precision of estimation. An operation of the dental technical fee automatic calculation system 100 according to Example 2 will be described with reference to the flowchart of FIG. 5.

S201: As in S101 of Example 1, an image data input unit 110 acquires image data. The image data of this example is assumed to be imprinted with a prosthesis and a labo slip.

The image data input unit 110 acquires information to be the basis of billing in the labo slip if features (barcode, QR code, document title, etc.) in the labo slip are recognized in an image. If the barcode, QR code, etc. are recognized, the image data input unit 110 acquires unique identification information contained in the barcode, QR code, and the like. Also, the image data input unit 110 acquires information (type of the prosthesis, quantity of the prosthesis, name of the material used, used material quantity, etc.) to be the basis of billing saved in association with the identification information from a management system or the like for a labo slip (not shown). Alternatively, if the information to be the basis of billing is described directly in the labo slip, the image data input unit 110 can read the basis of billing by using a known technology such as OCR.

Moreover, the image data input unit 110 extracts the feature quantity of the prosthesis from the image data, as in S101 of Example 1.

S202: As in S102 of Example 1, the image data input unit 110 inputs the feature quantity of the image data acquired in S101 to a learning unit 130. The learning unit 130 inputs the feature quantity of the image data to the learning model and obtains, as an output, basis data estimated to be highly correlated to the image data.

S203: A billing amount estimation unit 140 compares the basis data obtained from the learning model in S202 and the information to be the basis of billing obtained from the labo slip in S201. If both these items are coincident, the precision of the learning model can be assumed to be appropriate, so that the procedure transitions to S204. If these items are not coincident, the procedure transitions to S205 to improve the precision of the learning model.

S204: The billing amount estimation unit 140 assesses a billing amount based on the basis data obtained in S202 and a fee table retained in advance, as in S103 of Example 1.

S205: In order to maintain and improve the precision of the learning model, it is effective to add new data for learning to the learning model to update it. As typical update methods for the learning model, there are batch processing for newly remaking a learning model by giving past learning data and new learning data at a time and sequential learning (also called online learning) for sequentially updating an existing learning model by giving new learning data only. In this example, the learning model is updated by the online learning of which the load and time for calculation can be suppressed.

The image data input unit 110 outputs the feature quantity of the image data of the prosthesis acquired in S201 to the learning unit 130. Moreover, a basis data input unit 120 outputs, as basis data, information to be the basis of billing obtained from the labo slip in S201 to the learning unit 130. The learning unit 130 performs the online learning using a set of these image and basis data, thereby updating the learning model. Since specific processing for carrying out the online learning is a known technology as described in the following document, for example, a detailed description thereof is omitted herein.

Shai Shalev-Shwartz, Online Learning and Online Convex Optimization (Foundations and Trends in Machine Learning, 4(2): 107-194, 2011).

S206: The billing amount estimation unit 140 assesses the billing amount based on the information to be the basis of billing obtained from the labo slip in S201 and the fee table retained in advance, as in S103 of Example 1.

S207: The billing amount estimation unit 140 outputs the billing amount assessed in S204 or S206

<Example 3>

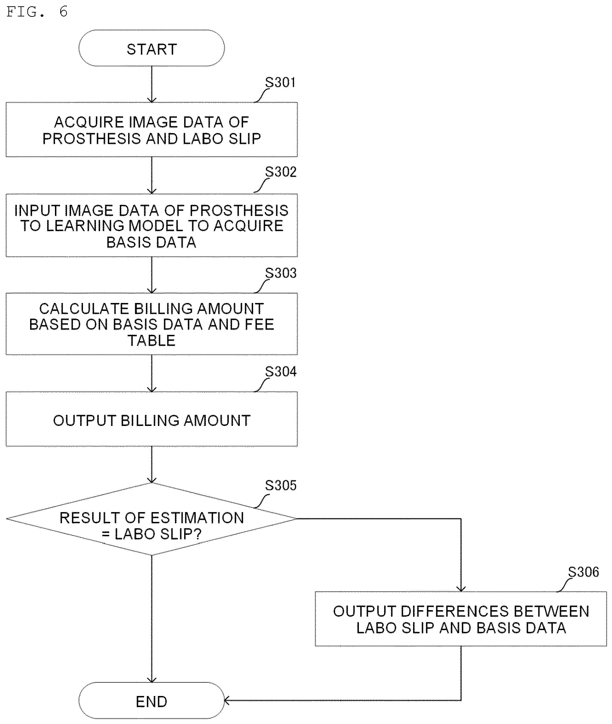

Example 3 relates to an automatic calculation system 100 capable of checking the accuracy of a labo slip using a learning model fully advanced in learning (i.e., having a sufficient estimation precision). An operation of the dental technical fee automatic calculation system 100 according to Example 3 will be described with reference to the flowchart of FIG. 6.

S301: As in S101 of Example 1, an image data input unit 110 acquires image data. The image data of this example is assumed to be imprinted with a prosthesis and a labo slip. As in S201 of Example 2, the image data input unit 110 acquires information to be the basis of billing in the labo slip.

S302: As in S102 of Example 1, the image data input unit 110 inputs the feature quantity of the image data acquired in S301 to a learning unit 130. The learning unit 130 inputs the feature quantity of the image data to the learning model and obtains, as an output, basis data assumed to be highly correlated to the image data.

S303: As in S103 of Example 1, a billing amount estimation unit 140 assesses a billing amount based on the basis data obtained in S302 and a fee table retained in advance.

S304: The billing amount estimation unit 140 outputs the billing amount assessed in S303.

S305: The billing amount estimation unit 140 compares the basis data obtained from the learning model in S302 and the information to be the basis of billing obtained from the labo slip in S301. If both these items are coincident, the contents of the labo slip can be assumed to be accurate. If these items are not coincident, the procedure transitions to S306.

S306: The billing amount estimation unit 140 outputs that part of the information to be the basis of billing obtained from the labo slip in S301 which is different from the basis data obtained from the learning model in S302. For example, different items can be displayed on a display device (not shown).

The present invention is not limited to the above-described embodiment and can be suitably changed without departing from the spirit of the invention. In the examples of the embodiment described above, the basis data constituting the basis of billing is input to the basis data input unit 120, and the learning unit 130 learns the correlation between the image data and the basis data. However, the present invention is not limited to this. For example, the billing amount may be input to the basis data input unit 120. In this case, the learning unit 130 learns the correlation between the image data and the billing amount in the learning mode. In the determination mode, moreover, the learning unit 130 outputs the billing amount corresponding to the prosthesis concerned when it is given the image data of the prosthesis as an input.

According to this technique, the dental technical fee automatic calculation system 100 can output the billing amount without comprising the billing amount estimation unit 140.

Furthermore, in the above-described embodiment, all the basis data constituting the basis of billing for the prosthesis are output in the learning mode by the basis data input unit 120. The correlation between the image data and all the input basis data is learned by the learning unit 130. However, the present invention is not limited to this. More specifically, the basis data input unit 120 may output only some of the basis data constituting the basis of billing for the prosthesis in the learning mode. The learning unit 130 may learn the correlation between the image data and the input some data.

If the basis of billing can be divided into a plurality of basis data groups A, B, C ··· (each of the basis data groups A, B, C ··· may include one or a plurality of basis data), for example, the learning unit 130 may construct each of learning models a, b, and c in the learning mode. The learning models a, b, and c indicate the correlation between the image data and the basis data group A, correlation between the image data and the basis data group B, and correlation between the image data and the basis data group C, respectively. In this case, the billing amount estimation unit 140 calculates the billing amount by the same technique as in the above-described embodiment after the basis data estimated by the learning unit 130 in the estimation mode, using the learning models a, b, and c, individually, are put together.

According to this technique, the dental technical fee automatic calculation system 100 can tune, reconstruct, or replace only those learning models related to specific basis data, as required. In this case, there is the advantage that those learning models related to the other basis data continue to be available.

Moreover, although the learning unit 130 is designed to learn the correlation between the image data and the basis data by supervised learning in the embodiment described above, the learning may alternatively be performed by another machine learning technique such as unsupervised learning or reinforcement learning.

Furthermore, each processing means constituting the present invention may be either composed of hardware or configured to logically implement any processing by urging a CPU (central processing unit) to execute a computer program. In this case, the computer program can be stored by using non-transitory computer readable media of various types and supplied to a computer. Also, the program may be supplied to the computer by transitory computer readable media of various types. The transitory computer readable media include electrical signals, optical signals, and electromagnetic waves. The transitory computer readable media can supply the program through wired communication paths, such as electric wires and optical fibers, or wireless communication paths.

[Reference Signs List]

100    DENTAL TECHNICAL FEE AUTOMATIC CALCULATION SYSTEM
110    IMAGE DATA INPUT UNIT
120    BASIS DATA INPUT UNIT
130    LEARNING UNIT
131    ERROR CALCULATION UNIT
132    MODEL UPDATE UNIT
140    BILLING AMOUNT ESTIMATION UNIT

**Claims**

1.  A dental technical fee automatic calculation system comprising:

    an image data input unit configured to input image data of a prosthesis;
    a basis data input unit configured to input basis data as a basis of assessment of a billing amount for the prosthesis; and
    a learning unit to which the image data and the basis data are input to construct a learning model indicative of a correlation between the image data and the basis data.

2.  The dental technical fee automatic calculation system according to claim 1, wherein the learning unit receives the input of the image data and outputs the basis data highly correlated to the image data based on the learning model, further comprising a billing amount estimation unit configured to estimate the billing amount based on the basis data.

3.  The dental technical fee automatic calculation system according to claim 1, wherein the image data input unit inputs the image data of the prosthesis and image data related to a labo slip, and the learning unit updates the learning model using the contents of the labo slip if the basis data output by the learning unit based on the image data of the basis data and the contents of the labo slip are different.

4.  The dental technical fee automatic calculation system according to claim 1, wherein the image data input unit inputs the image data of the prosthesis and image data related to a labo slip, and the billing amount estimation unit outputs the different items.

5.  A dental technical fee automatic calculation method comprising:

    an image data input step in which a computer inputs image data of a prosthesis;
    a basis data input step for inputting basis data as a basis of assessment of a billing amount for the prosthesis; and
    a learning step in which the image data and the basis data are input to construct a learning model indicative of a correlation between the image data and the basis data.

6.  he dental technical fee automatic calculation method according to claim 5, wherein the image data is input and the basis data highly correlated to the image data is output based on the learning model in the learning step, and the billing amount is estimated based on the basis data.

7.  The dental technical fee automatic calculation method according to claim 5, wherein the image data of the prosthesis and image data related to a labo slip are input in the image data input step, further comprising a step for updating the learning model using the contents of the labo slip if the basis data output based on the image data of the basis data in the learning step and the contents of the labo slip are different.

8. The dental technical fee automatic calculation method according to claim 5, wherein image data of the prosthesis and image data related to a labo slip are input in the image data input step, further comprising a step for outputting the different items if the basis data output based on the image data of the basis data in the learning step and the contents of the labo slip are different.

9. A program for urging a computer to execute the method according to any one of claims 5 to 8.

10. A dental technical fee automatic calculation system comprising:

an image data input unit configured to input image data of a prosthesis;
a basis data input unit configured to input a billing amount for the prosthesis; and
a learning unit to which the image data and the billing amount are input to construct a learning model indicative of a correlation between the image data and the billing amount.

FIG. 1

100

DENTAL TECHNICAL FEE AUTOMATIC CALCULATION SYSTEM

130

LEARNING
UNIT

110

IMAGE DATA
INPUT UNIT

IMAGE DATA

140

BILLING
AMOUNT
ESTIMATION
UNIT

120

BASIS DATA
INPUT UNIT

BASIS DATA

FIG. 2

100

DENTAL TECHNICAL FEE AUTOMATIC CALCULATION SYSTEM

130

LEARNING UNIT

131

ERROR
CALCULATION UNIT

TRAINING DATA: T
ERROR: E

110

IMAGE DATA
INPUT UNIT

IMAGE
DATA

140

BILLING
AMOUNT
ESTIMATION
UNIT

120

BASIS DATA
INPUT UNIT

BASIS
DATA

132

MODEL UPDATE
UNIT

CORRELATION
MODEL: M

FIG. 3

```
        ┌─────────────────┐
        │      START      │
        └─────────────────┘
                 │
                 ▼                          ⌐ S101
   ┌──────────────────────────────────────┐
   │   ACQUIRE IMAGE DATA OF PROSTHESIS    │
   └──────────────────────────────────────┘
                 │
                 ▼                          ⌐ S102
   ┌──────────────────────────────────────┐
   │   INPUT IMAGE DATA OF PROSTHESIS TO   │
   │  LEARNING UNIT TO ACQUIRE BASIS DATA  │
   └──────────────────────────────────────┘
                 │
                 ▼                          ⌐ S103
   ┌──────────────────────────────────────┐
   │   CALCULATE BILLING AMOUNT BASED ON   │
   │        BASIS DATA AND FEE TABLE       │
   └──────────────────────────────────────┘
                 │
                 ▼                          ⌐ S104
   ┌──────────────────────────────────────┐
   │        OUTPUT BILLING AMOUNT          │
   └──────────────────────────────────────┘
                 │
                 ▼
        ┌─────────────────┐
        │       END       │
        └─────────────────┘
```

FIG. 4

## FEE TABLE

| MATERIAL NAME | UNIT PRICE |
|---|---|
| P | 100 |
| Q | 150 |
| R | 200 |

| PROSTHESIS NAME | DENTAL TECHNICAL FEE FOR CREATION |
|---|---|
| A | 1000 |
| B | 2500 |
| C | 3000 |

FIG. 5

```
                    ( START )
                        │
                        │ S201
                        ▼
        ┌───────────────────────────────┐
        │     ACQUIRE IMAGE DATA OF      │
        │   PROSTHESIS AND LABO SLIP     │
        └───────────────────────────────┘
                        │
                        │ S202
                        ▼
        ┌───────────────────────────────┐
        │ INPUT IMAGE DATA OF PROSTHESIS │
        │  TO LEARNING MODEL TO ACQUIRE  │
        │           BASIS DATA           │
        └───────────────────────────────┘
                        │
                        │ S203
                        ▼
              ╱─────────────────╲
             ╱  RESULT OF ESTIMATION ╲──────────────────┐
             ╲    = LABO SLIP?       ╱                   │
              ╲─────────────────╱                        │
                        │                                │
                        │ S204                           │ S205
                        ▼                                ▼
        ┌───────────────────────────────┐   ┌───────────────────────────────┐
        │    CALCULATE BILLING AMOUNT    │   │  PERFORM SEQUENTIAL LEARNING   │
        │  BASED ON BASIS DATA AND FEE   │   └───────────────────────────────┘
        │            TABLE               │                   │
        └───────────────────────────────┘                   │ S206
                        │                                    ▼
                        │                    ┌───────────────────────────────┐
                        │                    │    CALCULATE BILLING AMOUNT    │
                        │                    │  BASED ON LABO SLIP AND FEE    │
                        │ S207               │            TABLE               │
                        ▼                    └───────────────────────────────┘
        ┌───────────────────────────────┐                   │
        │      OUTPUT BILLING AMOUNT     │◄──────────────────┘
        └───────────────────────────────┘
                        │
                        ▼
                    (  END  )
```

FIG. 6

```
                    ┌─────────────────┐
                    │      START      │
                    └────────┬────────┘
                             │              S301
                    ┌────────▼────────────────┐
                    │   ACQUIRE IMAGE DATA OF  │
                    │  PROSTHESIS AND LABO SLIP│
                    └────────┬─────────────────┘
                             │              S302
                    ┌────────▼─────────────────┐
                    │ INPUT IMAGE DATA OF       │
                    │ PROSTHESIS TO LEARNING    │
                    │ MODEL TO ACQUIRE          │
                    │ BASIS DATA                │
                    └────────┬─────────────────┘
                             │              S303
                    ┌────────▼─────────────────┐
                    │  CALCULATE BILLING AMOUNT │
                    │  BASED ON BASIS DATA AND  │
                    │  FEE TABLE                │
                    └────────┬─────────────────┘
                             │              S304
                    ┌────────▼─────────────────┐
                    │   OUTPUT BILLING AMOUNT   │
                    └────────┬─────────────────┘
```

S305 — RESULT OF ESTIMATION = LABO SLIP?

S306 — OUTPUT DIFFERENCES BETWEEN LABO SLIP AND BASIS DATA

END

## INTERNATIONAL SEARCH REPORT

| International application No. |
| --- |
| PCT/JP2017/043670 |

A. CLASSIFICATION OF SUBJECT MATTER
Int. Cl. G06Q50/22(2018.01)i

According to International Patent Classification (IPC) or to both national classification and IPC

B. FIELDS SEARCHED

Minimum documentation searched (classification system followed by classification symbols)
Int. Cl. G06Q10/00-99/00, A61C13/00-13/38, A61F2/00-2/80

Documentation searched other than minimum documentation to the extent that such documents are included in the fields searched
Published examined utility model applications of Japan    1922-1996
Published unexamined utility model applications of Japan  1971-2018
Registered utility model specifications of Japan          1996-2018
Published registered utility model applications of Japan  1994-2018

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| A | JP 2017-550 A (THE UNIVERSITY OF TOKYO) 05 January 2017, entire text, all drawings (Family: none) | 1-10 |
| A | JP 2012-33161 A (ROBERT REID INC.) 16 February 2012, entire text, all drawings (Family: none) | 1-10 |
| A | JP 2004-522489 A (NOBEL BIOCARE AB (PUBL)) 29 July 2004, entire text, all drawings & AU 2002217690 B2 & BR 01116644 A & CA 2432953 A1 & EP 1347713 A1 & US 2004/078212 A1 & WO 2002/053056 A1 | 1-10 |
| A | JP 2007-156706 A (FUJITSU LTD.) 21 June 2007, entire text, all drawings (Family: none) | 1-10 |

☒ Further documents are listed in the continuation of Box C.  ☐ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 07.02.2018 | 20.02.2018 |

| Name and mailing address of the ISA/ | Authorized officer |
| --- | --- |
| Japan Patent Office 3-4-3, Kasumigaseki, Chiyoda-ku, Tokyo 100-8915, Japan | Telephone No. |

Form PCT/ISA/210 (second sheet) (January 2015)

15

**INTERNATIONAL SEARCH REPORT**

International application No.

PCT/JP2017/043670

C (Continuation). DOCUMENTS CONSIDERED TO BE RELEVANT

| Category* | Citation of document, with indication, where appropriate, of the relevant passages | Relevant to claim No. |
|---|---|---|
| A | JP 2002-197197 A (MASTERWORKS KK) 12 July 2002, entire text, all drawings (Family: none) | 1-10 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016071784 A

**Non-patent literature cited in the description**

- *Foundations and Trends in Machine Learning,* 2011, vol. 4 (2), 107-194